# EUROPÄISCHE PATENTSCHRIFT

(11) **EP 2 802 294 B1**
(45) Veröffentlichungstag und Bekanntmachung des Hinweises auf die Patenterteilung: **18.05.2016**
(21) Anmeldenummer: 13712699.1
(22) Anmeldetag: 06.03.2013
(51) Int. Cl.: A61B 17/84, A61B 17/72, A61B 17/86, A61F 2/42

(54) **FUßCHIRURGISCHE INTRAMEDULLÄRE VERRIEGELUNGS- KNOCHENSCHRAUBE ZUR FIXATION DES GROßZEHENGRUNDGELENKS**
INTRAMEDULAR LOCKING BONE SCREW FOR FIXATION OF THE BIG-TOE JOINT
VIS DE VERROUILLAGE INTRAMÉDULLAIRE D'OSTÉOSYNTHÈSE EN CHIRURGIE DU PIED POUR LA FIXATION DE L'ARTICULATION BASALE DU GROS ORTEIL

(30) Priorität: 08.03.2012 DE 102012101978
(43) Veröffentlichungstag der Anmeldung: 19.11.2014
(73) Patentinhaber: Zimmer GmbH, 8404 Winterthur (CH)
(72) Erfinder: KOTULJAC, Vladko, 72355 Schömberg (DE); VITEK, Michael, A-1120 Wien (AT)
(74) Vertreter: Manitz, Finsterwald & Partner GbR
(86) Internationale Anmeldenummer: PCT/EP2013/054532
(87) Internationale Veröffentlichungsnummer: WO 2013/131974

(56) Entgegenhaltungen:
- EP-A1- 1 378 205
- WO-A1-2004/014243
- WO-A1-2004/043277
- DE-A1- 19 857 279
- US-A1- 2007 233 123
- US-A1- 2011 082 508
- US-B1- 6 306 140

## Beschreibung

Die Erfindung betrifft eine fußchirurgische intramedulläre Verriegelungs-Knochenschraube zur Fixation des Großzehengrundgelenks gemäß Anspruch1, welche sich entlang einer Längsachse erstreckt, mit einem eine erste Gewindesteigung aufweisenden ersten Außengewindeabschnitt und mit mindestens einem eine zweite, von der ersten Gewindesteigung unterschiedliche Gewindesteigung aufweisenden, vorzugsweise selbstfurchend ausgebildeten, zweiten Außengewindeabschnitt.
Bisher wurden zur Großzehengrundgelenksarthrodese Knochenplatten eingesetzt, die von außen mit Phalanges und Metatarsale verschraubt wurden. Aufgrund der hohen Belastung des Großzehengrundgelenks des ersten Strahls kann es bei der bekannten Lösung zu Deformationen der Knochenplatte und damit zu verzögerten Heilungsprozessen sowie Fehlstellungen kommen.

Aus der DE 28 07 364 A1 ist allgemein eine als Kompressionsschraube ausgebildete Knochenschraube bekannt, die zwei Außengewindeabschnitte mit voneinander unterschiedlicher Gewindesteigung aufweist. Die bekannte Knochenschraube ist nicht speziell für die Fußchirurgie konzipiert und weist das Problem einer nicht dauerhaft definierten Positionierung auf, was insbesondere im Fußbereich aufgrund der auftretenden Belastungen problematisch ist.
Aus der EP 1 354 562 A1 ist ein Knochenfixierungssystem, umfassend eine als Kopfschraube ausgebildete und damit nicht vollständig im Knochen aufnehmbare Schraube mit einem axial durchgehenden Außengewinde gleichbleibender Steigung bekannt, wobei eine Knochenkompression nur aufwändig von außen erreichbar ist, indem der Kopf der Knochenschraube auf einen Teil der Fraktur gedrückt wird, während das Gewinde der Schraube im anderen Teil der Fraktur eingeschraubt ist. Hier ist die Kompressionswirkung mangelhaft. Darüber hinaus kommt es aufgrund des Schraubenkopfes zu Weichteilreizungen und dadurch bedingt möglicherweise zu einem schlechteren Heilungserfolg. Die bekannte Knochenschraube weist seitliche Fixierkanäle auf, die allesamt auf dem gleichen Umfangswinkel angeordnet sind.

Aus der WO 2008/003433 A1 ist ein Implantat zur Osteosynthese einer basisnahen Osteotomie eines Mittelfußknochens bekannt. Um mit dem bekannten, außengewindefreien Implantat eine Kompressionswirkung erreichen zu können, ist es im vorderen Bereich mit in Umfangsrichtung versetzt angeordneten Fixierkanälen zur Aufnahme von Fixierschrauben versehen, wobei die Fixierschrauben hier lediglich die Haltefunktion des Gewindes einer Schraube übernehmen, während die Kompression an anderer Stelle durch eine Querschraube erreicht wird.

Die EP 1 378 205 A1 offenbart eine Knochenschraube nach dem Oberbegriff des Anspruchs 1.

Die US 5,964,768 B offenbart eine Knochenschraube mit einer kontinuierlich variierenden Gewindesteigung und einem vollständig konusförmigen Kern.

Ausgehend von dem Stand der Technik liegt der Erfindung die Aufgabe zugrunde, ein verbessertes Implantat zur Großzehengrundgelenksarthrodese des ersten Strahls anzugeben, welches sich durch eine hohe Robustheit auszeichnet. Insbesondere sollen vorstehend erläuterte Nachteile vermieden werden.

Diese Aufgabe wird mit einer fußchirurgischen intramedullären Verriegelungs-Knochenschraube mit den Merkmalen des Anspruchs 1 gelöst. Vorteilhafte Weiterbildungen der Erfindung sind in den Unteransprüchen angegeben.

Der Erfindung liegt der Gedanke zugrunde, zur Fixation des Großzehengrundgelenks im menschlichen Fuß eine intramedullär anordnenbare Verriegelgungs-Knochenschraube anzugeben, die sich durch mindestens zwei Außengewindeabschnitte mit einer unterschiedlichen Steigung auszeichent, um eine Kompression zwischen Phalanges und Metatarsale zu erreichen. Darüber hinaus ist erfindungsgemäß vorgesehen, dass die Knochenschraube mindestens einen die Schraube winklig, vorzugsweise rechtwinklig, zur Längsachse durchsetzenden Fixierkanal aufweist, in welchen von außen Fixiermittel einbringbar sind, insbesondere eine Fixierschraube einschraubbar ist, um die Knochenschraube in der gewünschten Einbringposition fixieren und gegen Verdrehen sichern zu können.

Bevorzugt ist die Axialerstreckung der Knochenschraube aus einem Wertebereich zwischen 40 und 60mm gewählt. Ganz besonders bevorzugt beträgt die Axialerstreckung etwa 50mm. Besonders zweckmäßig ist es zudem, wenn der maximale Außendurchmesser der Knochenschraube weniger als 15mm, vorzugsweise weniger als 12mm beträgt. Ganz besonders bevorzugt ist der maximale Außendurchmesser aus einem Wertebereich zwischen 6mm und 11 mm vorzugsweise zwischen 7 und 10mm gewählt. Noch weiter bevorzugt beträgt er etwa 9mm.

Die erfindungsgemäße Knochenschraube wird bevorzugt bei den Indikationen Hallux rigidus Stadien III und IV sowie massiv abweichender Hallux valgus verwendet.

Besondere Vorteile des Einsatzes der nach dem Konzept der Erfindung ausgebildeten Knochenschraube sind, dass es im Gegensatz zu den bisher verwendeten Knochenplatten keine Weichteilprobleme gibt, da die Knochenschraube vollständig im Knochen aufgenommen ist. Nach der Operation ist eine sofortige Vollbelastung im Komfortschuh möglich. Darüber hinaus resultiert eine enorm feste Kompressionsarthrodese, die aufgrund der Verriegelung mit Hilfe der Fixiermittel nicht die Kompression verliert woraus eine rasche Knochenheilung resultiert.

Erfindungsgemäß ist vorgesehen, zusätzlich zu dem ersten Fixierkanal mindestens einen zweiten, die Knochenschraube durchsetzenden Fixierkanal zur Aufnahme von weiteren Fixiermitteln, insbesondere einer (weiteren) Fixierschraube vorzusehen, um die Knochenschraube an mindestens zwei axial beabstandeten Positionen durch Einschrauben einer seitlichen Fixierschraube fixieren zu können und/oder um während der Operation eine größere Flexibilität zu haben und nicht auf eine einzige Fixierposition beschränkt zu sein. Als besonders zweckmäßig hat es sich erwiesen, wenn mindestens zwei Fixierkanäle nicht parallel ausgerichtet sind, sondern in Umfangsrichtung versetzte Ein- und Ausgänge haben. Anders ausgedrückt ist es bevorzugt, wenn mindestens zwei Fixierkanäle um einen Winkel, von vorzugsweise 90°, verdreht zueinander angeordnet sind, um eine Fixierung der Knochenschraube aus unterschiedlichen Seitenrichtungen zu ermöglichen.

Dabei sind der erste und der zweite, in Umfangsrichtung zueinander versetzt angeordnete Fixierkanal auf den ersten und den zweiten Außengewindeabschnitt verteilt angeordnet. Mit anderen Worten befindet sich der erste Fixierkanal im zweiten Außengewindeabschnitt und der dazu in Umfangsrichtung versetzt angeordnete zweite Fixierkanal im ersten, vorzugsweise hinteren Außengewindeabschnitt 6. Die Verteilung der in Umfangsrichtung versetzt angeordneten Fixierkanäle auf die beiden eine voneinander unterschiedliche Steigung aufweisenden Außengewindeabschnitte ermöglicht eine gute Erreichbarkeit der Fixierkanäle aus unterschiedlichen Umfangspositionen und gewährleistet eine optimale verdrehsichere Anordnung.

Die vollständige Einbringbarkeit der erfindungsgemäßen Knochenschraube in dem menschlichen Knochen wird vorzugsweise dadurch erreicht, dass die Schraube keinen gestuften Schraubenkopf aufweist. Vielmehr ist vorgesehen, dass sich eine Konuskontur eines Schraubenkerns ausgehend von einem zylindrischen Abschnitt bis an das axial hintere Knochenschraubenende erstreckt.
Im Falle des Vorsehens von mehr als zwei seitlichen Fixierkanälen, was bevorzugt ist, ist es besonders vorteilhaft, wenn mindestens zwei, vorzugsweise drei Fixierkanäle parallel zueinander angerichtet sind. Noch weiter bevorzugt ist es, wenn (bevorzugt ausschließlich) ein hinterster Fixierkanal in Umfangsrichtung, vorzugsweise um 90°, zu anderen Fixierkanälen verdreht ist, noch weiter bevorzugt zu sämtlichen anderen Fixierkanälen.

Um ein zielgerichtetes Einbringen der Knochenschraube in die Knochen des ersten Strahl eines menschlichen Fußes zu ermöglichen ist es besonders zweckmäßig, wenn die Knochenschraube kanüliert ist, um einen Bohrdraht, vorzugsweise mit einem Durchmesser von 1,6mm aufnehmen zu können. Wobei es noch weiter bevorzugt ist, wenn der erste Fixierkanal und/oder der zweite Fixierkanal, vorzugsweise sämtliche Fixierkanäle den zentrischen Durchgangskanal, insbesondere unter einem Winkel von 90° schneidet/schneiden.
Um das Einbringen der Knochenschraube in die Fußknochen zu erleichtern und um zudem eine gute Fixierung bzw. einen guten Halt der Knochenschraube im ersten Strahl des menschlichen Fußes zu erreichen, ist es besonders zweckmäßig wenn der Außendurchmesser des ersten und des zweiten Außengewindes unterschiedlich groß ist, wobei es noch weiter bevorzugt ist, wenn der erste Außengewindeabschnitt hinter dem zweiten Außengewindeabschnitt, d.h. näher, vorzugsweise unmittelbar benachbart zu einem vorzugsweise vorgesehenen (hinteren) Antrieb der Knochenschraube vorgesehen ist.

Um eine optimale Kompression der miteinander zu verbindenden Knochensegmente/Knochenabschnitte zu gewährleisten ist es besonders zweckmäßig, wenn der Kerndurchmesser der Knochenschraube im Bereich des ersten Außengewindeabschnitts sich über die Längserstreckung der Knochen verändert, wobei es noch weiter bevorzugt ist, wenn der Kerndurchmesser sich in diesem Bereich von hinten nach vorne verjüngt, insbesondere konisch konturiert ist.

Noch weiter bevorzugt ist es, wenn die Hüllkontur des ersten Außengewindes nicht an die Kerndurchmesserveränderung angepasst ist, sondern über die Axialerstreckung, zumindest über den größten Teil der Axialerstreckung zylindrisch konturiert ist. Besonders zweckmäßig ist es, wenn der sich verändernde Kerndurchmesserbereich, insbesondere der konisch konturierte Bereich des Kerns einen hinteren Abschnitt der Schraube bildet bzw. in einem hinteren Bereich der Schraube angeordnet ist, wobei es noch weiter bevorzugt ist, wenn ein axial daran angrenzender Kerndurchmesserabschnitt zylindrisch konturiert ist. Bevorzugt beträgt die Axialerstreckung des zylindrisch konturierten Kerndurchmesserabschnittes ein Vielfaches, insbesondere ein Drei- bis Sechsfaches der Axialerstreckung des sich verändernden Kerndurchmesserabschnittes. Bevorzugt beträgt der Konuswinkel des konischen Abschnittes, gemessen zwischen einer Konusmantelfläche und der Längsmittelachse der Schraube zwischen etwa 2 und 6°, vorzugsweise beträgt er 4°.

Ganz besonders bevorzugt ist es, wenn der erste Außengewindedurchmesser im Bereich des einen Antrieb aufweisenden hinteren Knochenschraubenendes angeordnet ist und eine geringere Steigung aufweist als der, vorzugsweise über einen gewindefreien Axialabschnitt beabstandete, zweite Außengewindeabschnitt. Vorzugsweise weist der erste Außengewindeabschnitt insgesamt die geringste Steigung auf - bezogen auf sämtliche Außengewindeabschnitte. Als besonders zweckmäßig hat es sich erwiesen, wenn der erste Außengewindeabschnitt selbstfurchend ausgebildet ist.

Optimale Fixierungsergebnisse bzw. eine optimale Montageerleichterung wird erreicht, wenn zusätzlich zu dem ersten und dem zweiten Außengewindeabschnitt mindestens ein, vorzugsweise ausschließlich ein, dritter Außengewindeabschnitt vorgesehen ist, der sich durch mindestens ein Geometriemerkmal von den weiteren Außengewindeabschnitten (erster und zweiter Außengewindeabschnitt) unterscheidet. So ist es besonders zweckmäßig, wenn der dritte Außengewindeabschnitt einen geringeren Außendurchmesser aufweist als der erste und/oder der zweite Außengewindeabschnitt. Bevorzugt ist der Kerndurchmesser im Bereich des zweiten und dritten Außengewindeabschnitts konstant. Noch weiter bevorzugt ist es, wenn die Axialerstreckung des dritten Außengewindeabschnittes größer ist als die des ersten und/oder des zweiten Außengewindeabschnittes. Idealerweise ist der zweite Außengewindeabschnitt axial zwischen dem ersten und dem dritten Außengewindeabschnitt angeordnet, wobei noch weiter bevorzugt der dritte Außengewindeabschnitt einen vordersten Außengewindeabschnitt bildet. Dabei ist es besonders zweckmäßig, wenn axial vorne an den dritten Außengewindeabschnitt ein gewindefreier Axialabschnitt anschließt.

Ganz besonders bevorzugt ist die Steigung des ersten Außengewindeabschnittes aus einem Wertebereich zwischen 1,5 und 2mm gewählt und beträgt noch weiter bevorzugt 2mm. Ganz besonders bevorzugt ist die Steigung des zweiten Außengewindeabschnittes aus einem Wertebereich zwischen 2,0mm und 3,0mm gewählt und beträgt vorzugsweise ca. 2,4mm. Noch weiter bevorzugt ist die Steigung eines fakultativen dritten Außengewindeabschnittes aus einem Wertebereich zwischen 2,0mm und 3,0mm gewählt und beträgt vorzugsweise 2,4mm.

Ideal ist es, wenn zweiter und dritter Außengewindeabschnitt unmittelbar ineinander übergehen. Als besonders zweckmäßig hat es sich herausgestellt, wenn in jedem der drei Außengewindeabschnitte einer der vorerwähnten Fixierkanäle vorgesehen ist, wobei bevorzugt im ersten Außengewindeabschnitt zwei in Umfangsrichtung um einen Winkel, insbesondere von 90° zueinander verdrehte Fixierkanäle vorgesehen sind.

Um dem Operateur die Positionierung der Knochenschraube zu erleichtern, bzw. um die Fixierkanäle an definierte Umfangspositionen zu positionieren, ist in Weiterbildung der Erfindung mit Vorteil vorgesehen, dass an der hinteren Stirnseite der Knochenschraube eine entsprechende Markierung vorgesehen ist und zwar in Form eines sich in radialer Richtung erstreckenden, nach hinten offenen Querkanals, der eine zentrische hintere Antriebsvertiefung in seitlicher Richtung, d.h. in radialer Richtung bis zum Außenumfang der Knochenschraube öffnet, wobei der Querkanal vorzugsweise so ausgebildet ist, dass in diesen aus radialer Richtung von außen temporär Fixiermittel, insbesondere eine Fixierschraube oder ein Fixierstift eingebracht werden kann, um ein Zielgerät zum Einbringen der Fixiermittel in die Fixierkanäle relativ zu der Knochenschraube exakt zu einem vorzugsweise verwendeten Zielgerät ausrichten zu können, mit welchem Fixierschrauben in die vorzugsweise innengewindefreien Fixierkanäle eingebracht werden können.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
Fig. 1a
   bis
Fig. 1 e klapprichtige Darstellungen eines bevorzugten Ausführungsbeispiels einer intramedullären Verriegelungs-Knochenschraube zur Fixation des Großzehengrundgelenks im ersten Strahl,
Fig. 2 eine Schnittansicht entlang der Schnittlinie A-A gemäß Fig. 1 a, und
Fig. 3 eine perspektivische Schrägansicht von schräg hinten der in den vorstehenden Figuren gezeigten Knochenschraube.

In den Figuren sind gleiche Elemente mit der gleichen Funktion mit den gleichen Bezugszeichen gekennzeichnet.

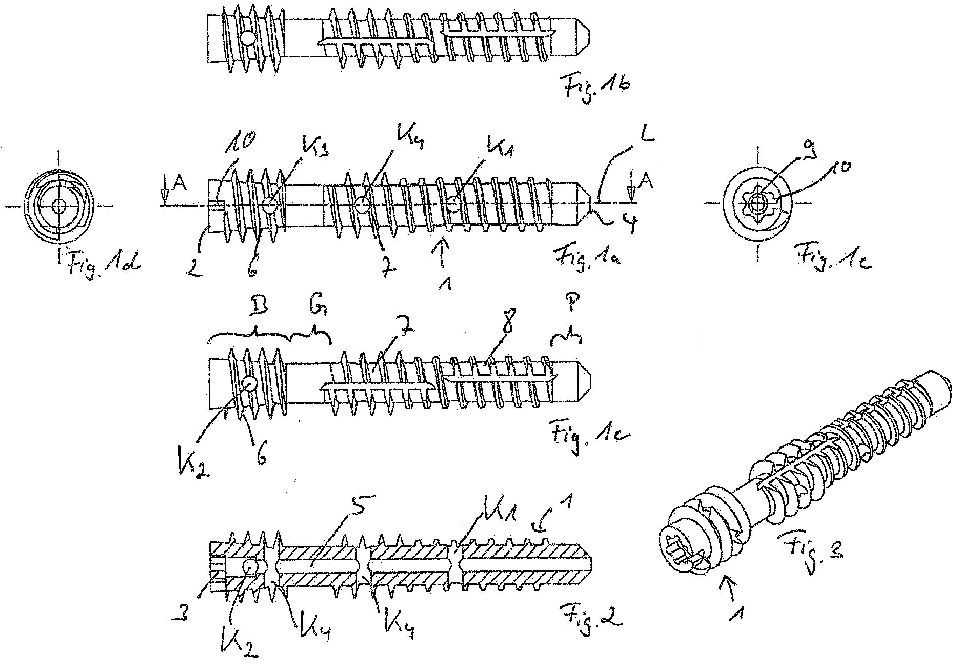

In den Fig. 1a bis 3 ist eine fußchirurgische intramedulläre Verriegelungs-Knochenschraube 1 zur Fixation des Großzehengrundgelenks gezeigt. Die Knochenschraube erstreckt sich von einem hinteren Ende 2 mit hier als Torx-Antrieb ausgebildeten Antrieb 3 entlang einer Längsachse L bis zu einem vorderen Ende 4. Wie sich aus der Schnittansicht gemäß Fig. 2 ergibt, ist die Knochenschraube 1 kanüliert ausgebildet und weist einen axial durchgehenden zentrischen Durchgangskanal 5 zur Aufnahme eines Kirschnerdrahtes, hier eines 1.6 Kirschnerdrahtes auf. In dem gezeigten Ausführungsbeispiel beträgt die Axialerstreckung der Knochenschraube 1 50mm.

In einen hinteren Bereich B verjüngt sich ein Kerndurchmesser der Schraube von 6,4mm auf 5mm, woraus ein Konuswinkel von etwa 4° resultiert. Der sich verjüngende Kerndurchmesserabschnitt erstreckt sich ausgehend vom hinteren Ende 2 10mm nach vorne. An diesen hinteren Bereich B schließt ein Bereich mit konstantem Kerndurchmesser an, der in etwa die vierfache Länge des hinteren Bereichs B aufweist. Die Knochenschraube 1 schließt vorne mit einem konisch angefassten vorderen Endbereich ab.

Im hinteren Bereich B der Knochenschraube 1 befindet sich ein erster, selbstfurchend ausgebildeter Außengewindeabschnitt 6, der eine zylindrische Hüllkontur aufweist. An diesen ersten Außengewindeabschnitt 6 schließt vorne ein gewindefreier Axialabschnitt G an, dessen Axialerstreckung etwa 5mm beträgt. An den gewindefreien Abschnitt G schließt ein zweiter, von dem ersten Außengewindeabschnitt 6 unterschiedlicher Außengewindeabschnitt 7 an, der axial übergeht in einen dritten Außengewindeabschnitt 8, an welchen axial ein weiterer, kurzer gewindefreier Abschnitt P anschließt. Sowohl der zweite als auch der dritte Außengewindeabschnitt 7, 8 sind selbstfurchend ausgebildet. Zu diesem Zweck sind entsprechende Schneidkanten vorgesehen, wobei die Schneidkanten des ersten, zweiten und dritten Außengewindeabschnittes in Umfangsrichtung versetzt angeordnet sind. Im gezeigten Ausführungsbeispiel beträgt der Außendurchmesser des ersten Außengewindeabschnittes 6 9mm. Dessen Steigung beträgt 2mm und die Axialerstreckung des ersten Außengewindeabschnittes beträgt 8mm.

Der zweite Außengewindeabschnitt 7 hat eine Axialerstreckung von 10mm. Der konstante Außendurchmesser beträgt 8mm und die Steigung 2,4mm. Der dritte Außengewindeabschnitt hat einen geringeren konstanten Außendurchmesser von 6,5mm.

Die Steigung entspricht der Steigung des zweiten Außengewindeabschnittes also in dem gezeigten Ausführungsbeispiel 2,4mm. Die Axialerstreckung entspricht in etwa der doppelten Axialerstreckung des zweiten Außengewindeabschnittes, in dem gezeigten Ausführungsbeispiel also 10mm.

Aus den unterschiedlichen Ansichten ist zu erkennen, dass mehrere senkrecht zur Längsachse L orientierte Fixierkanäle vorgesehen sind, nämlich ein erster Fixierkanal K1 im Bereich des dritten Außengewindeabschnittes 8, ein in Umfangsrichtung um 90° hierzu verdreht angeordneter zweiter Fixierkanal K2, der einen hintersten Fixierkanal bildet und im Bereich des ersten Außengewindeabschnittes 6 angeordnet ist. Zudem sind zwei weitere Fixierkanäle, nämlich ein dritter Fixierkanal K3, ebenfalls im Bereich des ersten Außengewindeabschnittes 6 und ein vierter Fixierkanal K4 im Bereich des zweiten Außengewindeabschnittes vorgesehen. Die Fixierkanäle K1, K3 und K4 sind parallel zueinander ausgerichtet, axial voneinander beabstandet, in dem gezeigten Ausführungsbeispiel gleichmäßig voneinander beabstandet.

Bemerkenswert ist eine hintere, zentrische, hier als Torx-Vertiefung ausgebildete Antriebsvertiefung 9, die nicht umfangsgeschlossen ausgebildet ist, sondern über einen Querkanal 10 seitlich geöffnet ist, welcher nach hinten offen ausgebildet ist. Über diesen Querkanal 10 kann ein Zielgerät exakt zur Knochenschraube 1 ausgerichtet werden, um die Knochenschraube durch Einbringen von mindestens einer Fixierschraube in mindestens einen der Fixierkanäle K1 bis K4 verdrehsicher bzw. axial Fixieren zu können. Bevorzugt erfolgt die Fixierung im ersten Strahl des Fußes wie folgt: Zunächst wird ein Bohrdraht an der Phalanges in das Metatarsale I plantar eingebracht.

Dieser Bohrdraht wird dann, vorzugsweise mit einem 6mm kanülierten Bohrer überbohrt, woraufhin die Knochenschraube 1 über den zentralen Bohrdraht eingedreht wird. Die bevorzugte Montage eines Zielgerätes erfolgt sodann, wobei das Ausrichten zur Knochenschraube 1 über einen seitlichen Stift erfolgt, der von radial außen in den Querkanal 10 eingebracht wird.

### Bezugszeichenliste

- 1: Knochenschraube
- 2: hinteres Ende
- 3: Antrieb
- 4: vorderes Ende
- 5: Durchgangskanal
- 6: erster Außengewindeabschnitt
- 7: zweiter Außengewindeabschnitt
- 8: dritter Außengewindeabschnitt
- 9: Antriebsvertiefung
- 10: Querkanal

- B: hinterer Bereich
- G: gewindefreier Abschnitt
- L: Längsachse
- P: gewindefreier Axialabschnitt
- K1: erster Fixierkanal
- K2: zweiter Fixierkanal
- K3: dritter Fixierkanal
- K4: vierter Fixierkanal

## Patentansprüche

1. Vollständig im Knochen aufnehmbare, fußchirurgische intramedulläre Verriegelungs-Knochenschraube zur Fixation des Großzehengrundgelenks, die sich entlang einer Längsachse (L) erstreckt, mit einem eine erste Gewindesteigung aufweisenden ersten Außengewindeabschnitt (6) und mit mindestens einem eine zweite, von der ersten Gewindesteigung unterschiedliche Gewindesteigung aufweisenden zweiten Außengewindeabschnitt (7),
wobei mindestens ein sich winklig zur Längsachse (L) erstreckender und die Knochenschraube (1) durchsetzender erster Fixierkanal (K1) zur Aufnahme von Fixiermitteln im zweiten Außengewindeabschnitt (7) vorgesehen ist,
wobei ein Kerndurchmesser der Knochenschraube (1) im Bereich des ersten Außengewindeabschnittes (6) sich über die Längserstreckung der Knochenschraube (1) verändert, nämlich sich von hinten nach vorne verjüngt,
wobei der Kerndurchmesser der Knochenschraube (1) im Bereich des ersten Außengewindeabschnittes (6) konisch konturiert ist, wobei sich die Konusform des Kerns ausgehend von einem zylindrischen Abschnitt bis an das axiale hintere Knochenschraubenende fortsetzt,
**dadurch gekennzeichnet, dass**
ein mit Axialabstand zum ersten Fixierkanal (K1) im ersten Außengewindeabschnitt (6) angeordneter und die Knochenschraube (1) durchsetzender zweiter Fixierkanal (K2) zur Aufnahme von Fixiermitteln vorgesehen ist,
wobei eine erste Längsmittelachse des ersten Fixierkanals (K1) in Umfangsrichtung der Knochenschraube (1) um einen Winkel verdreht angeordnet ist zu einer zweiten Längsmittelachse des zweiten Fixierkanals (K2),
wobei der erste Außengewindeabschnitt (6) im Bereich des einen Antrieb (3) aufweisenden hinteren Knochenschraubenendes angeordnet ist und eine geringere Steigung aufweist als der zweite Außengewindeabschnitt (7),
wobei der Konuswinkel des konischen Abschnitts des Kerns, gemessen zwischen einer Konusmantelfläche und der Längsachse (L), zwischen etwa 2 und 6°, vorzugsweise 4°, beträgt, und
wobei der erste Außengewindeabschnitt (6) eine zylindrische Hüllkontur aufweist.

2. Knochenschraube nach Anspruch 1,
**dadurch gekennzeichnet,**
**dass** die Knochenschraube (1) keinen abgestuften Schraubenkopf aufweist.

3. Knochenschraube nach einem der vorhergehenden Ansprüche, **gekennzeichnet,**
**durch** einen eine dritte Längsmittelachse aufweisenden, die Knochenschraube (1) durchsetzenden Fixierkanal (K1-K4) zur Aufnahme von Fixiermitteln, wobei die dritte Längsmittelachse vorzugsweise parallel zur Längserstreckung des ersten oder zweiten Fixierkanals (K1, K2) angeordnet ist.

4. Knochenschraube nach einem der vorhergehenden Ansprüche,
**dadurch gekennzeichnet,**
**dass** die Knochenschraube (1) eine sich entlang der Längsachse (L) erstreckenden Durchgangskanal (5) zur Aufnahme eines Kirschnerdrahtes aufweist.

5. Knochenschraube nach einem der vorhergehenden Ansprüche,
**dadurch gekennzeichnet,**
**dass** zumindest der erste Fixierkanal (K1), vorzugsweise sämtliche Fixierkanäle (K1-K4) den Durchgangskanal (5) rechtwinklig schneidet/schneiden.

6. Knochenschraube nach einem der vorhergehenden Ansprüche,
**dadurch gekennzeichnet,**
**dass** der Außendurchmesser des ersten und des zweiten Außengewindeabschnittes (7,8) unterschiedlich ist.

7. Knochenschraube nach einem der vorhergehenden Ansprüche,
**dadurch gekennzeichnet,**
**dass** ein, vorzugsweise selbstfurchend ausgebildeter, dritter Außengewindeabschnitt (8) vorgesehen ist, der vorzugsweise einen geringeren Außendurchmesser aufweist als der erste und/oder zweite Außengewindeabschnitt (6,7) und/oder dessen Steigung der Steigung des zweiten Außengewindeabschnittes (7) entspricht und/oder dessen Axialerstreckung größer ist als die des ersten und/oder zweiten Außengewindeabschnittes (6,7) und/oder der den zweiten Außengewindeabschnitt (7) mit dem ersten Außengewindeabschnitt (6) axial zwischen sich aufnimmt und/oder der unmittelbar in den zweiten Außengewindeabschnitt (7) übergeht.

8. Knochenschraube nach einem der vorhergehenden Ansprüche,
**dadurch gekennzeichnet,**
**dass** die Knochenschraube (1) im Bereich des hinteren Endes (2) eine zentrische Antriebsvertiefung (9) aufweist, insbesondere eine Torx-Antriebsvertiefung, und dass die Antriebsvertiefung (9) an, vorzugsweise ausschließlich, einer Stelle über einen axial nach hinten offenen Querkanal (10) mit einer Außenumfangsseite der Knochenschraube (1) verbunden ist.

9. Knochenschraube nach einem der vorhergehenden Ansprüche,
**dadurch gekennzeichnet,**
**dass** der zweite Außengewindeabschnitt (7) über einen gewindefreien Axialabschnitt (G) vom ersten Gewindeabschnitt (6) beabstandet ist.

10. Knochenschraube nach einem der vorhergehenden Ansprüche,
**dadurch gekennzeichnet,**
**dass** der zweite Außengewindeabschnitt (7) selbstfurchend ausgebildet ist.

11. Knochenschraube nach einem der vorhergehenden Ansprüche,
**dadurch gekennzeichnet,**
**dass** der erste Fixierkanal (K1) sich rechtwinklig zur Längsachse (L) erstreckt, und/oder dass die erste Längsmittelachse des ersten Fixierkanals (K1) in Umfangsrichtung der Knochenschraube (1) um einen Winkel von 90° verdreht angeordnet ist zu der zweiten Längsmittelachse des zweiten Fixierkanals (K2).

## Claims

1. An intramedullary locking bone screw for fixing the metatarsophalangeal joint of the big toe in foot surgery which is completely receivable in the bone and which extends along a longitudinal axis (L), having a first outer threaded section (6) having a first thread pitch and having at least one second outer threaded section (7) having a second thread pitch different from the first thread pitch,
wherein at least one first fixing passage (K1) is provided for the reception of fixing means in the second outer threaded section (7), the passage extending at an angle with respect to the longitudinal axis (L) and passing through the bone screw (1);
wherein a core diameter of the bone screw (1) in the region of the first outer threaded section (6) changes over the longitudinal extent of the bone screw (1), namely tapers from the rear to the front;
wherein the core diameter of the bone screw (1) in the region of the first outer threaded section (6) is conically contoured,
wherein the conical shape of the core continues starting from a cylindrical section up to the axial rear end of the bone screw,
**characterized in that**
a second fixing passage (K2) for the reception of fixing means is provided which has an axial spacing with respect to the first fixing passage (K1), which is arranged in the first outer threaded section (6) and which passes through the bone screw (1);
wherein a first longitudinal middle axis of the first fixing passage (K1) is arranged rotated by an angle with respect to a second longitudinal middle axis of the second fixing passage (K2) in the circumferential direction of the bone screw (1);
wherein the first outer threaded section (6) is arranged in the region of a rear bone screw end having a drive (3) and has a smaller pitch than the second outer threaded section (7);
wherein the conical angle of the conical section of the core, measured between a conical jacket surface and the longitudinal axis (L), amounts to approximately between 2 and 6°, and preferably amounts to 4°; and wherein the first outer threaded section (6) has a cylindrical sleeve contour.

2. A bone screw in accordance with claim 1,
**characterized in that**
the bone screw (1) does not have a stepped screw head.

3. A bone screw in accordance with any one of the preceding claims,
**characterized by**
a fixing passage (K1-K4) for the reception of fixing means which passes through the bone screw (1) and which has a third longitudinal middle axis, wherein the third longitudinal middle axis is preferably arranged in parallel to the longitudinal extent of the first or second fixing passage (K1, K2).

4. A bone screw in accordance with any one of the preceding claims,
**characterized in that**
the bone screw (1) has a through passage (5) for the reception of a Kirschner wire, with the through passage extending along the longitudinal axis (L).

5. A bone screw in accordance with any one of the preceding claims,
**characterized in that**
at least the first fixing passage (K1), preferably all fixing passages (K1-K4), cuts/cut the through passage (5) at right angles.

6. A bone screw in accordance with any one of the preceding claims,
**characterized in that**
the outer diameter of the first and the second outer threaded sections (7, 8) is different.

7. A bone screw in accordance with any one of the preceding claims,
**characterized in that**
a third outer threaded section (8) is provided, preferably configured in a self-tapping manner, which preferably has a smaller outer diameter than the first and/or the second outer threaded sections (6, 7) and/or whose pitch corresponds to the pitch of the second outer threaded section (7) and/or whose axial extent is larger than that of the first and/or the second outer threaded sections (6, 7) and/or which axially receives the second outer threaded section (7) with the first outer threaded section (6) between itself and/or which directly transitions into the second outer threaded section (7).

8. A bone screw in accordance with any one of the preceding claims,
**characterized in that**
the bone screw (1) has a central drive recess (9) in the region of the rear end (2), in particular a Torx drive recess; and **in that** the drive recess (9) is connected at a position to an outer circumferential side of the bone screw (1) via an axially backwardly open transverse passage (10), with the drive recess preferably being exclusively connected to the outer circumferential side of the bone screw (1).

9. A bone screw in accordance with any one of the preceding claims,
**characterized in that**
the second outer threaded section (7) is spaced apart from the first threaded section (6) by a thread-free axial section (G).

10. A bone screw in accordance with any one of the preceding claims,
**characterized in that**
the second outer threaded section (7) is configured in a self-tapping manner.

11. A bone screw in accordance with any one of the preceding claims,
**characterized in that**
the first fixing passage (K1) extends at a right angle with respect to the longitudinal axis (L) and/or **in that** the first longitudinal middle axis of the first fixing passage (K1) is arranged rotated by an angle of 90° with respect to the second longitudinal middle axis of the second fixing passage (K2) in the circumferential direction of the bone screw (1).

## Revendications

1. Vis à os de verrouillage intramédullaire en chirurgie du pied, capable d'être logée entièrement dans l'os, pour la fixation de l'articulation basale du gros orteil, qui s'étend le long d'un axe longitudinal (L), avec un premier tronçon à filetage extérieur (6) présentant un premier pas de filetage, et avec au moins un second tronçon à filetage extérieur (7) présentant un second pas de filetage différent du premier pas de filetage,
dans laquelle il est prévu au moins un canal de fixation (K1), qui s'étend sous un angle par rapport à l'axe longitudinal (L) et qui traverse la vis à os (1), pour la réception d'organes de fixation dans le second tronçon à filetage extérieur (7),
dans laquelle un diamètre de coeur de la vis à os (1) dans la région du premier tronçon de filetage extérieur (6) varie sur l'extension longitudinale de la vis à os (1), à savoir qu'il va en se rétrécissant de l'arrière vers l'avant,
dans laquelle le diamètre de coeur de la vis à os (1) présente un contour conique dans la région du premier tronçon à filetage extérieur (6),
dans laquelle la forme conique du coeur se poursuit depuis un tronçon cylindrique jusqu'à l'extrémité axiale postérieure de la vis à os, **caractérisée en ce que**
il est prévu un second canal de fixation (K2), agencé à une distance axiale du premier canal de fixation (K1) dans le premier tronçon à filetage extérieur (6) et traversant la vis à os (1), pour la réception d'organes de fixation,
dans laquelle un premier axe médian longitudinal du premier canal de fixation (K1) est agencé tourné d'un angle en direction périphérique de la vis à os (1) par rapport à un second axe médian longitudinal du second canal de fixation (K2),
dans laquelle le premier tronçon à filetage extérieur (6) est agencé dans la région de l'extrémité arrière de la vis à os comportant un entraînement (3), et présente un pas plus faible que le second tronçon à filetage extérieur (7),
dans laquelle l'angle du tronçon conique du noyau, mesuré entre une surface enveloppe conique et l'axe longitudinal (L), est compris entre environ 2 et 6°, de préférence 4°, et
dans laquelle le premier tronçon à filetage extérieur (6) présente un contour enveloppe cylindrique.

2. Vis à os selon la revendication 1,
**caractérisée en ce que** la vis à os (1) ne comprend aucune tête de vis en gradins.

3. Vis à os selon l'une des revendications précédentes, **caractérisée par** un canal de fixation (K1-K4), présentant un troisième axe médian longitudinal et traversant la vis à os (1), pour la réception d'organes de fixation, dans laquelle le troisième axe médian longitudinal est agencé de préférence parallèlement à l'extension longitudinale du premier ou du second canal de fixation (K1, K2).

4. Vis à os selon l'une des revendications précédentes,
**caractérisée en ce que** la vis à os (1) comporte un canal traversant (5) s'étendant le long de l'axe longitudinal (L), pour la réception d'un fil de Kirschner.

5. Vis à os selon l'une des revendications précédentes,
**caractérisée en ce qu'**au moins le premier canal de fixation (K1) et de préférence la totalité des canaux de fixation (K1-K4) recoupe(nt) le canal traversant (5) à angle droit.

6. Vis à os selon l'une des revendications précédentes,
**caractérisée en ce que** le diamètre extérieur du premier et du second tronçon à filetage extérieur (7, 8) est différent.

7. Vis à os selon l'une des revendications précédentes,
**caractérisée en ce qu'**il est prévu un troisième tronçon à filetage extérieur (8) réalisé de préférence de manière autoforeuse, qui présente un diamètre extérieur plus faible que le premier et/ou le second tronçon à filetage extérieur (6, 7) et/ou dont le pas correspond au pas du second tronçon à filetage extérieur (7), et/ou dont l'extension axiale est plus grande que celle du premier et/ou du second tronçon à filetage extérieur (6, 7) et/ou qui reçoivent axialement entre eux le second tronçon à filetage extérieur (7) avec le premier tronçon à filetage extérieur (6), et/ou qui se transforme directement dans le second tronçon à filetage extérieur.

8. Vis à os selon l'une des revendications précédentes,
**caractérisée en ce que** la vis à os (1) comporte, dans la région de l'extrémité postérieure (2), un renfoncement d'entraînement centré (9), en particulier un renfoncement d'entraînement du type torx, et **en ce que** le renfoncement d'entraînement (9) est relié à un côté périphérique extérieur de la vis à os à un emplacement, et de préférence exclusivement un emplacement, via un canal transversal (10) ouvert axialement vers l'arrière.

9. Vis à os selon l'une des revendications précédentes,
**caractérisée en ce que** le second tronçon à filetage extérieur (7) est écarté du premier tronçon à filetage (6) par un tronçon axial (G) dépourvu de filetage.

10. Vis à os selon l'une des revendications précédentes,
**caractérisée en ce que** le second tronçon à filetage extérieur (7) est réalisé de manière autoforeuse.

11. Vis à os selon l'une des revendications précédentes,
**caractérisée en ce que** le premier canal de fixation (K1) s'étend à angle droit par rapport à l'axe longitudinal (L), et/ou **en ce que** le premier axe médian longitudinal du premier canal de fixation (K1) est agencé tourné d'un angle de 90° en direction périphérique de la vis à os (1) par rapport au second axe médian longitudinal du second canal de fixation (K2).
